# EUROPÄISCHE PATENTSCHRIFT

(11) **EP 2 358 457 B1**
(45) Veröffentlichungstag und Bekanntmachung des Hinweises auf die Patenterteilung: **08.01.2014**
(21) Anmeldenummer: 09764228.4
(22) Anmeldetag: 26.11.2009
(51) Int. Cl.: B01D 27/08, B01D 35/30

(54) **WECHSELFILTERELEMENT**
INTERCHANGEABLE FILTER ELEMENT
ELÉMENT DE FILTRE INTERCHANGEABLE

(30) Priorität: 26.11.2008 DE 202008015675 U
(43) Veröffentlichungstag der Anmeldung: 24.08.2011
(73) Patentinhaber: Mann + Hummel GmbH, 71638 Ludwigsburg (DE)
(72) Erfinder: LOOS, Rainer, 71691 Freiberg (DE); LUKA, Helmut, Marbach, 71672 (DE); EBERLE, Jürgen, 72076 Tübingen (DE); PFLÜGER, Frank, 74343 Sachsenheim (DE)
(86) Internationale Anmeldenummer: PCT/EP2009/065866
(87) Internationale Veröffentlichungsnummer: WO 2010/060942

(56) Entgegenhaltungen:
- FR-A1- 2 911 797

## Beschreibung

### Technisches Gebiet

Die Erfindung betrifft ein Wechselfilterelement für den stehenden Einbau im Ölkreislauf insbesondere eines Kraftfahrzeuges der im Oberbegriff des Anspruchs 1 angegebenen Gattung.

### Stand der Technik

Im Ölkreislauf von Kraftfahrzeugmotoren, aber auch von anderen Motoren wie stationären Brennkraftmaschinen werden Ölfilter mit einem Wechselfilterelement eingesetzt, wobei das Wechselfilterelement in bestimmten Wartungsintervallen auszutauschen ist. Beim sogenannten stehenden Einbau wird das Wechselfilterelement in Schwerkraftrichtung von oben eingesetzt bzw. nach oben ausgebaut. Das Filtergehäuse des Wechselfilterelementes weist hierzu eine in Schwerkraftrichtung untere Stirnwand mit einem umlaufenden Dichtring auf, dessen Anlagefläche im montierten Zustand an einer Dichtfläche des systemfesten Filterteils dichtend anliegt. Das systemfeste Filterteil umfasst den Zulauf und den Ablauf für das durch das Wechselfilterelement hindurchgeleitete Öl. Beispielsweise offenbart die FR 2 911 797 A1 ein Wechselfilterelement für den stehenden Einbau.

In verbreiteter Bauform ist an der Stirnwand des Wechselfilterelementes ein zentraler Anschlussstutzen befestigt, der im montierten Zustand mit dem Zulauf und dem Ablauf in Wechselwirkung steht. Insbesondere kann eine Ablaufsteuerung am systemfesten Filterteil vorgesehen sein, welche durch den zentralen Anschussstutzen betätigt wird. Bei Entnahme des Wechselfilterelementes wird die Ablaufsteuerung geöffnet, so dass aus dem Wechselfilterelement austretendes Öl vom systemfesten Filterteil aufgefangen und abgeleitet wird.

Beim Lösen des Wechselfilterelementes hebt sich als erstes der Dichtring von seinem Dichtsitz ab, während der zentrale Anschlussstutzen die Ablaufsteuerung immer noch geschlossen hält. Erst bei weiterer Entnahme des Wechselfilterelementes wird auch die Ablaufsteuerung geöffnet, so dass sie ihrer Funktion nachkommen kann. Zwischen dem Abheben des Dichtringes von seinem Dichtsitz und dem Öffnen der Ablaufsteuerung verbleibt ein Entnahmeweg, in dem einerseits die Dichtwirkung aufgehoben und andererseits die Ablaufsteuerung noch nicht in Betrieb genommen ist. Die Verbindungsstelle zwischen dem als separates Teil ausgeführten Anschlussstutzen und der Stirnwand des Filtergehäuses ist im Regelfall nicht öldicht. Bei der Entnahme des gebrauchten und noch mit Öl gefüllten Wechselfilterelementes kann durch diese Verbindungsstelle Öl austreten. In der vorgenannten Zwischenposition wird das austretende Öl nicht aufgefangen und abgeleitet, so dass eine Verschmutzung des Motorraumes eintreten kann.

Der vorliegenden Erfindung liegt die Aufgabe zugrunde, ein Wechselfilterelement der eingangs genannten Gattung derart weiterzubilden, dass ein Ölaustritt beim Ausbau des gebrauchten Wechselfilterelements vermieden ist.

Diese Aufgabe wird durch ein Wechselfilterelement mit den Merkmalen des Anspruchs 1 gelöst.

### Offenbarung der Erfindung

Es wird eine Weiterbildung des gattungsgemäßen Wechselfilterelementes derart vorgeschlagen, dass am Dichtring radial innenseitig seiner Anlagefläche eine umlaufende Dichtlippe einteilig angeformt ist, die dichtend an einer Dichtfläche des Anschlussstutzens anliegt. Die Verbindungsstelle zwischen dem Anschlussstutzen und der Stirnwand des Filtergehäuses liegt hierbei auf der Öl führenden Innenseite des Wechselfilterelementes. Demnach verhindert die Dichtlippe einen Ölaustritt im Bereich dieser Verbindungsstelle. Das im Inneren des Filtergehäuses angesammelte Altöl kann lediglich durch den Anschlussstutzen ablaufen. Dies ist jedoch erst dann möglich, wenn das Wechselfilterelement so weit herausgeschraubt ist, dass die zugehörige Ablaufsteuerung des systemfesten Filterteils geöffnet ist. Das in diesem Zustand aus dem Wechselfilterelement austretende Altöl wird vollständig von der Ablaufsteuerung aufgenommen. Ein seitlicher Ölaustritt und eine Verschmutzung des Motorraumes sind zuverlässig vermieden.

In bevorzugter Weiterbildung ist der zentrale Anschlussstutzen mittels Rastlaschen in eine mittige Öffnung der Stirnwand des Filtergehäuses eingerastet, wobei die Rastlaschen vollständig von der Dichtlippe übergriffen sind. Die einzelnen Rastlaschen sind zwar durch öldurchlässige Schlitze voneinander getrennt. Da diese jedoch vollständig von der Dichtlippe umschlossen sind, ist hier ein unerwünschter Ölaustritt zuverlässig vermieden. Gleichzeitig ist eine einfache und effiziente Montage sowie sichere Halterung des Anschlussstutzens am Filtergehäuse möglich.

Es kann zweckmäßig sein, dass die umlaufende Dichtlippe an einer axialen bzw. stirnseitigen Dichtfläche des Anschlussstutzens anliegt. Bevorzugt ist die Dichtfläche eine umlaufende Umfangsfläche des Anschlussstutzens, an der die Dichtlippe radial anliegt. Hierdurch tritt die Dichtwirkung unabhängig von axialen Lagetoleranzen des Anschlussstutzens relativ zum Filtergehäuse ein, wodurch eine zuverlässige Dichtwirkung sichergestellt ist.

Es kann zweckmäßig sein, die Dichtlippe radial innenseitig der Anlagefläche des Dichtringes anzuordnen. Bevorzugt steht die Dichtlippe axial über die Anlagefläche des Dichtringes hervor. Dies erfordert in der radialen Richtung ein geringes Bauvolumen des Dichtringes und lässt gleichzeitig einen großen radialen dichtenden Federweg der Dichtlippe zu.

Der Dichtring des Wechselfilterelementes kann als Radialdichtung ausgeführt sein. Bevorzugt ist er eine Axialdichtung, wobei seine Anlagefläche stirnseitig des Dichtringes angeordnet ist. Bei einfachem Aufbau sowie leichter Montierbarkeit bzw. Demontierbarkeit ist eine zuverlässige Dichtwirkung erzielbar.

### Kurze Beschreibung der Zeichnungen

Ein Ausführungsbeispiel der Erfindung ist nachstehend anhand der Zeichnung näher erläutert. In der Zeichnung zeigt:
Fig. 1 in perspektivischer Darstellung ein erfindungsgemäßes Wechselfilterelement mit einem Anschlussstutzen und einem Dichtring, dessen Dichtlippe an der Umfangswand des Anschlussstutzens anliegt,
Fig. 2 in Längsschnittdarstellung den Anschlussstutzen nach Fig. 1 mit Einzelheiten seiner Rastlaschen,
Fig. 3 in perspektivischer Ansicht den Dichtring nach Fig. 1 mit Einzelheiten seiner daran angeformten Dichtlippe,
Fig. 4 in Längsschnittdarstellung das Wechselfilterelement nach Fig. 1 mit

Details zur gegenseitigen Wechselwirkung seiner Bauteile.

### Ausführungsform(en) der Erfindung

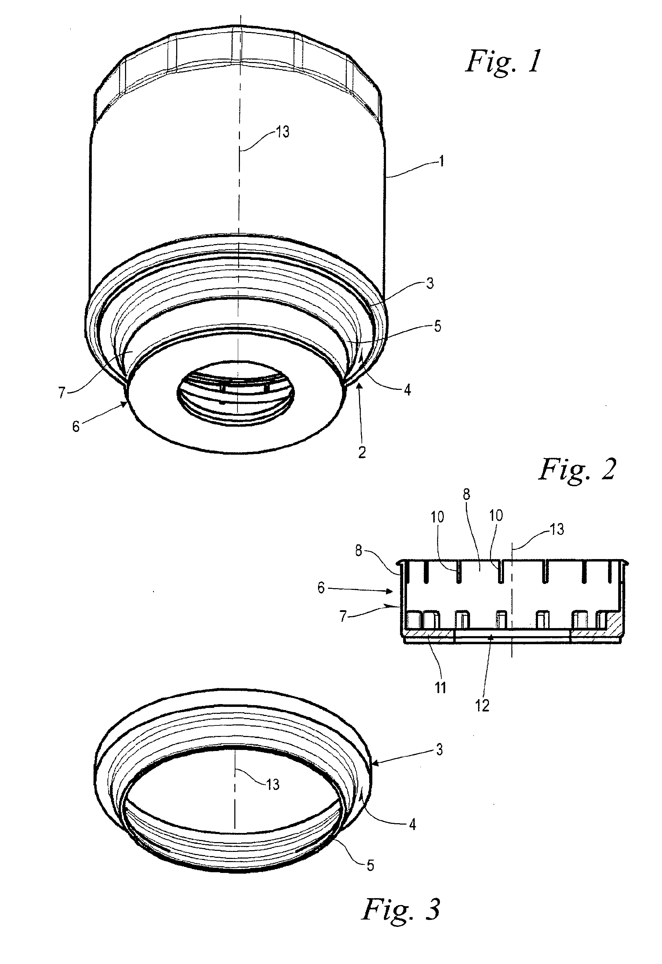

Fig. 1 zeigt in perspektivischer Darstellung ein erfindungsgemäßes Wechselfilterelement mit einem Filtergehäuse 1, einem Dichtring 3 und einem zentralen Anschlussstutzen 6. Das Wechselfilterelement ist im Wesentlichen rotationssymmetrisch zu einer Längsachse 13 aufgebaut. Das Filtergehäuse 1 weist eine Stirnwand 2 auf, an der der umlaufende Dichtring 3 mit einer Anlagefläche 4 angeordnet ist. Der zentrale Anschlussstutzen 6 ist zentrisch zur Längsachse 13 radial innenseitig des Dichtringes 3 derart an der Stirnwand 2 befestigt, dass der Dichtring 3 um den Anschlussstutzen 6 herumläuft.

Der Anschlussstutzen 6 weist eine umlaufende zylindrische Dichtfläche 7 auf. Am Dichtring 3 ist radial innenseitig seiner Anlagefläche 4 eine umlaufende Dichtlippe 5 angeformt, die dichtend an der Dichtfläche 7 des Anschlussstutzens 6 anliegt.

Fig. 2 zeigt in Längsschnittdarstellung den Anschlussstutzen 6 nach Fig. 1. Der Anschlussstutzen 6 ist topfförmig ausgestaltet, wobei eine umlaufende zylindrische Wand mit der Dichtfläche 7 von einem quer zur Längsachse 13 liegenden Boden 11 ausgeht. Im Boden 11 ist mittig zur Längsachse 13 eine Öffnung 12 vorgesehen. Im Bereich ihrer dem Boden 11 gegenüberliegenden Kante ist die Umfangswand des Anschlussstutzens 5 abseits der Dichtfläche 7 in Form einer Vielzahl von Rastlaschen 8 ausgebildet, die in der Umfangsrichtung durch Spalte 10 voneinander getrennt sind.

Fig. 3 zeigt in perspektivischer Ansicht den Dichtring 3 nach Fig. 1. Es ist zu erkennen, dass der Dichtring 3 als Axialdichtung ausgestaltet ist, dessen Anlagefläche 4 stirnseitig de Dichtringes 3 angeordnet ist. Die ebenfalls zentrisch um die Längsachse 13 umlaufende Dichtlippe 5 ist einteilig am Dichtring 3 angeformt. Dabei liegt sie radial innenseitig der Anlagefläche 4 und steht axial über die Anlagefläche 4 hervor.

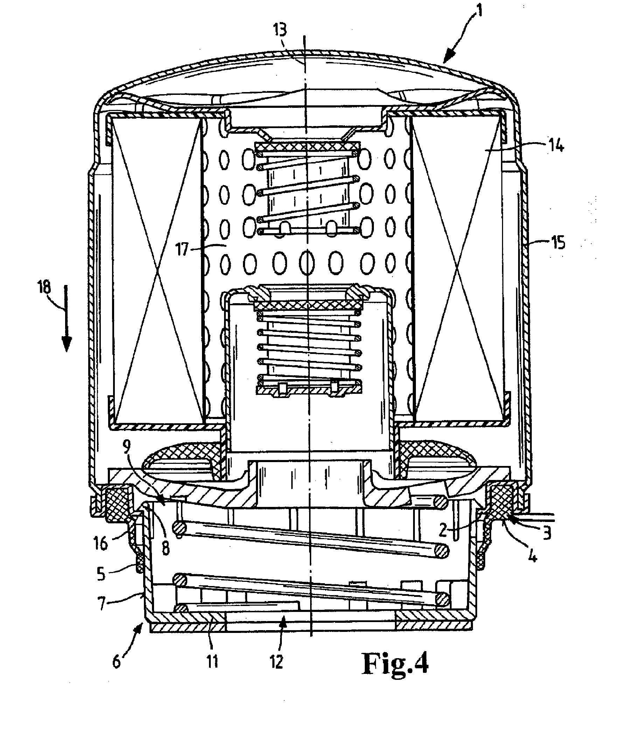

Fig. 4 zeigt in Längsschnittdarstellung das Wechselfilterelement nach Fig. 1 mit Details zur gegenseitigen Wechselwirkung seiner Bauteile. Das Wechselfilterelement ist für den stehenden Einbau im Motor-Ölkreislauf insbesondere eines Kraftfahrzeuges vorgesehen. Eine in gewöhnlicher Montageposition wirkende Gewichtskraft ist durch einen Pfeil 18 angegeben. Demnach liegen die Stirnwand 2, der Dichtring 3 mit der Dichtlippe 5 und der Anschlussstutzen 6 in der vorgesehenen Einbaulage bezogen auf die Gewichtskraftrichtung 18 unten.

Das Filtergehäuse 1 umfasst einen nach unten offenen Gehäusetopf 15, in dessen offene Seite die Stirnwand 2 eingesetzt ist. In die Stirnwand 2 ist im Umfangsbereich eine umlaufende Nut eingeformt, in der der umlaufende Dichtring 3 mit seinem Rechteckquerschnitt gehalten ist. Die Anlagefläche 4 des Dichtringes 3 liegt in der axialen Richtung frei. Im montierten Zustand ist die Anlagefläche 4 gegen eine Dichtfläche eines hier nicht dargestellten systemfesten Filterteils angedrückt, wodurch der Innenraum des Filtergehäuses 1 gegen das systemfeste Filterteil öldicht abgedichtet ist.

Im Innenraum des Filtergehäuses 1 ist ein zentraler Träger 17 angeordnet, auf dem ein Filtereinsatz 14 gehalten ist. Im montierten und mittels des Dichtringes 3 abgedichteten Zustand wird das Wechselfilterelement bzw. dessen Filtereinsatz 14 mit Öl durchströmt, wobei der Innenraum des Filtergehäuses 1 mit Öl gefüllt ist. Zu- und Ablauf des Öles erfolgen durch die Öffnung 12 im Boden 11 des Anschlussstutzens 6 sowie durch eine mittige Öffnung 9 in der Stirnwand 2 des Filtergehäuses 1.

Die mittige Öffnung 9 der Stirnwand 2 ist durch eine umlaufende Kante 16 begrenzt. Die Rastlaschen 8 des Anschlussstutzens 6 greifen hinter die Kante 16, wodurch der Anschlussstutzen 6 mit der Stirnwand 2 verrastet ist. Die zum Einrasten erforderliche Nachgiebigkeit der Rastlaschen 8 ist durch die Spalte 10 (Fig. 2) sichergestellt. Die Dichtlippe 5 liegt radial außenseitig an der Dichtfläche 7 des Anschlussstutzens 6 an. In der axialen Richtung übergreift sie ausgehend von der Stirnwand 2 die Rastlaschen 8 und die zwischenliegende Spalte 10 (Fig. 2) derart vollständig, dass die Rastlaschen 8 und die Spalte 10 in ihrer Gesamtheit auf der Öl führenden Innenseite des Filtergehäuses 1, des Dichtringes 3, der Dichtlippe 5 und der Dichtfläche 7 liegen. Die einteilige Anformung der Dichtlippe 5 am Dichtring 3 verhindert, dass Öl aus dem Filtergehäuse 1 im Anschlussbereich des Anschlussstutzens 6 an der Stirnwand 2 und insbesondere durch die Spalte 10 (Fig. 2) austritt. Bei der Entnahme des Wechselfilterelementes kann Öl aus dem Innenraum des Filtergehäuses 1 lediglich durch die Öffnungen 9, 12 austreten.

Der Boden 11 des Anschlussstutzens 6 steht in Wechselwirkung mit einer nicht dargestellten Ablaufsteuerung eines ebenfalls nicht dargestellten systemfesten Filterteils. Bei der Entnahme des Wechselfilterelementes entgegen der Gewichtskraftrichtung 18 gibt der Boden 11 die Ablaufsteuerung frei, so dass aus der Öffnung 12 austretendes Öl aufgefangen und abgeleitet wird. Ein seitlicher Ölaustritt ist durch die Dichtlippe 5 verhindert, so dass eine Motorraumverschmutzung auch dann zuverlässig vermieden ist, wenn die Anlagefläche 4 des Dichtringes 3 schon von ihrem Dichtsitz abgehoben ist, ohne aber dass die Ablaufsteuerung bereits durch den Boden 11 des Anschlussstutzens 6 freigegeben ist.

Alternativ zu der hier gezeigten Ausgestaltung sind aber auch abweichende Bauformen mit gleicher Wirkung möglich. So kann es beispielhaft vorteilhaft sein, dass der Dichtring 3 als Radialdichtung ausgestaltet ist. Die Dichtlippe 5 kann auch zweckmäßig an einer stirnseitigen Dichtfläche des Anschlussstutzens 6 in axialer Richtung anliegen. Außerdem kann es vorteilhaft sein, die Dichtlippe 5 radial innenseitig des Dichtringes 3 ohne axialen Versatz zur Anlagefläche 4 anzuordnen.

## Patentansprüche

1. Wechselfilterelement für den stehenden Einbau im Ölkreislauf, insbesondere eines Kraftfahrzeuges, umfassend ein Filtergehäuse (1) mit einer Stirnwand (2), an der ein umlaufender Dichtring (3) mit einer Anlagefläche (4) angeordnet ist, wobei ein zentraler Anschlussstutzen (6) an der Stirnwand (2) befestigt ist, **dadurch gekennzeichnet, dass** das am Dichtring (3) radial innenseitig seiner Anlagefläche (4) eine umlaufende Dichtlippe (5) einteilig angeformt ist, die dichtend an einer Dichtfläche (7) des Anschlussstutzens (6) anliegt.

2. Wechselfilterelement nach Anspruch 1, **dadurch gekennzeichnet, dass** der zentrale Anschlussstutzen (6) mittels Rastlaschen (8) in eine mittige Öffnung (9) der Stirnwand (2) eingerastet ist, wobei die Rastlaschen (8) vollständig von der Dichtlippe (5) übergriffen sind.

3. Wechselfilterelement nach Anspruch 1 oder 2, **dadurch gekennzeichnet, dass** die Dichtfläche (7) eine umlaufende Umfangsfläche des Anschlussstutzens (6) ist.

4. Wechselfilterelement nach einem der Ansprüche 1 bis 3, **dadurch gekennzeichnet, dass** die Dichtlippe (5) axial über die Anlagefläche (4) des Dichtringes (3) hervorsteht.

5. Wechselfilterelement nach einem der Ansprüche 1 bis 4, **dadurch gekennzeichnet, dass** der Dichtring (3) eine Axialdichtung ist, wobei die Anlagefläche (4) stirnseitig des Dichtringes (3) angeordnet ist.

## Claims

1. Spin-on filter element for the upright installation in the oil-circulation system, in particular for a motor vehicle, comprising a filter housing (1) with an end wall, on which a circumferential seal ring (3) with a contact area (4) is disposed, a central connecting piece (6) being attached to the end wall (2), **characterized in that** at the seal ring (3) radially inside of its contact area (4) a circumferential sealing lip (5) is integrally molded in one piece, which sealingly contacts the sealing surface (7) of the connecting piece (6).

2. Spin-on filter element according to claim 1, **characterized in that** the central connecting piece (6) is locked in the central opening (9) of the end wall (2) by means of locking latches (8), the locking latches (8) being completely overlapped by the sealing lip (5).

3. Spin-on filter element according to claim 1 or 2, **characterized in that** the sealing surface (7) is a circumferential peripheral surface of the connecting piece (6).

4. Spin-on filter element according to one of the claims 1 to 3, **characterized in that** the sealing lip (5) axially projects the contact area (4) of the seal ring (3).

5. Spin-on filter element according to one of the claims 1 to 4, **characterized in that** the seal ring (3) is an axial seal, the contact area being frontally disposed of the seal ring (3).

## Revendications

1. Élément de filtre interchangeable pour le montage vertical dans le circuit d'huile, notamment d'un véhicule automobile, comprenant un boîtier de filtre (1) avec une paroi frontale sur laquelle est disposée une bague d'étanchéité (3) circulaire avec une surface de contact (4), une tubulure de raccord (6) centrale étant fixée sur la paroi frontale (2), **caractérisé en ce qu'**une lèvre d'étanchéité (5) circulaire est formée en un seul bloc sur la bague d'étanchéité (3), en sens radial, à l'intérieur de sa surface de contact (4) et que cette lèvre est appliquée de façon étanche sur une surface d'étanchéité (7) de la tubulure de raccord (6).

2. Élément de filtre interchangeable selon la revendication 1, **caractérisé en ce que** la tubulure de raccord (6) centrale est encliquetée par des languettes d'encliquetage (8) dans une ouverture (9) centrale de la paroi frontale (2), les languettes d'encliquetage (8) étant totalement recouvertes par la lèvre d'étanchéité (5).

3. Élément de filtre interchangeable selon la revendication 1 ou 2, **caractérisé en ce que** la surface d'étanchéité (7) est une surface circonférentielle circulaire de la tubulure de raccord (6).

4. Élément de filtre interchangeable selon l'une des revendications 1 à 3, **caractérisé en ce que** la lèvre d'étanchéité (5) dépasse en sens axial de la surface de contact (4) de la bague d'étanchéité (3).

5. Élément de filtre interchangeable selon l'une des revendications 1 à 4, **caractérisé en ce que** la bague d'étanchéité (3) est un joint axial, la surface de contact étant disposée à l'avant de la bague d'étanchéité (3).
